# EUROPEAN PATENT SPECIFICATION

(11) **EP 3 727 309 B1**
(45) Date of publication and mention of the grant of the patent: **14.09.2022**
(21) Application number: 18825641.6
(22) Date of filing: 14.12.2018
(51) Int. Cl.: A61K 8/44, A61K 8/46, A61Q 5/02, A61K 8/73, A61K 8/81, A61K 8/892

(54) **PERSONAL CLEANSING COMPOSITIONS**
KÖRPERREINIGUNGSZUSAMMENSETZUNGEN
COMPOSITION D'HYGIÈNE PERSONNELLE LIQUIDE

(30) Priority: 21.12.2017 EP 17209538
(43) Date of publication of application: 28.10.2020
(73) Proprietor: Unilever IP Holdings B.V., 3013 AL Rotterdam (NL); Unilever Global IP Limited, Wirral, Merseyside CH62 4ZD (GB)
(72) Inventor: MOGHADAM, Arash, Mohajer, Bebington, Wirral Merseyside CH63 3JW (GB); MUSCAT, Joseph, Bebington, Wirral Merseyside CH63 3JW (GB)
(74) Representative: Chisem, Janet
(86) International application number: PCT/EP2018/085074
(87) International publication number: WO 2019/121428

(56) References cited:
- WO-A1-2007/065537
- WO-A1-2016/085707
- WO-A1-2017/071915
- WO-A1-2018/007332

## Description

### Field of the Invention

The present invention relates to shampoo compositions for use on hair.

### Background and Prior Art

US 2012/276210 relates to shampoo compositions containing polyacrylate microcapsules, wherein the polyacrylate microcapsules have increased deposition onto hair. A shampoo composition is disclosed comprising:
(a) from about 0.001% to about 10% of an anionic charged polyacrylate microcapsule;
(b) from about 0.01% to about 2% of a cationic deposition polymer; and
(c) from about 2% to about 25% of a detersive surfactant; and
(d) a carrier.

WO 2007/065537 addresses a problem associated with the use of cationic deposition polymers in that it is difficult to obtain a good balance of conditioning benefits at different stages of the shampooing process, and discloses an aqueous shampoo composition comprising:
(i) one or more anionic cleansing surfactants;
(ii) discrete, dispersed droplets of a water-insoluble conditioning agent with a mean droplet diameter (D3,2) of 4 micrometres or less;
(iii) one or more cationic polymers (A) selected from cationically modified acrylamide polymers having a cationic charge density at pH7 of less than 1.0 meq per gram, cationically modified celluloses and mixtures thereof, and
(iv) one or more cationic polymers (B) selected from cationically modified acrylamide polymers having a cationic charge density at pH7 of greater than 1.0 meq per gram, cationically modified polygalactomannans, and mixtures thereof, wherein the composition comprises a cationic polymer other than a cationically modified acrylamide polymer.

WO 2013/122861 discloses a conditioning composition additive for providing immediate and prolonged benefit to a keratin surface comprising:
a) a hydrophobically modified poly(acrylamido-N-propyltrimethylammonium chloride) (polyAPTAC) and b) water;
wherein the hydrophobically modified polyAPTAC is present in an amount of from 0.1 wt % to 20 wt % of the total weight of the conditioning composition additive and has a cationic charge density in the range of about 1 to 8 meq/g.

WO2017/071915 discloses a personal cleansing composition comprising:
(i) one or more cleansing surfactants
(ii) emulsified silicone
(iii) encapsulated benefit agent and
(iv) a combination of a cationic polygalactomannan and an acrylaminopropyltrimonium chloride/ acrylamide copolymer.

Many cleansing and conditioning products for use on hair contain silicones. It is desirable to deposit silicone onto hair in order to confer conditioning and sensory benefits. A typical hair wash process involves first washing hair with a shampoo and rinsing, followed by applying a conditioner product and rinsing.

Silicone can be deposited onto hair from a shampoo. However, we have found that this silicone is largely deterged when the hair is subsequently washed with a conditioner as part of a typical washing process. A consequence of this is that it is necessary to include silicone in the conditioner in order to provide conditioning benefits that are apparent when the hair has dried.

We have now found that a shampoo comprising a homopolymer of (3-acrylamidopropyl) trimethyl ammonium chloride can enhance the adhesion of the silicone delivered from shampoo and help retain it on hair during and after washing with a conditioner.

When the hair is washed with a shampoo containing N-DurHance A1000 and then a silicone free conditioner, it has a significantly higher disposition of silicone compared to hair that is washed with a 1% silicone containing shampoo and then a silicone free conditioner.

In dry friction data, the hair that is washed with a shampoo containing N-DurHance A1000 and then a silicone free conditioner has a significantly lower dry friction compared to the hair that is washed with a 1% silicone containing shampoo and then a silicone free conditioner.

### Summary of the Invention

In a first aspect the present invention provides an aqueous shampoo for hair comprising:-
a. a pre-formed emulsified silicone;
b. a cationic deposition polymer;
c. a hair substantive cationic conditioning polymer which is a homopolymer of (3-acrylamidopropyl) trimethyl ammonium chloride;
d. a cleansing surfactant;
e. a co-surfactant; and
f. a suspending agent.

In a second aspect, the present invention provides a method of treating hair comprising the steps of applying to hair the composition of the first aspect and performing a first rinse with water.

Silicone is deposited onto the hair from the composition of the invention, during the method of the invention.

Preferably, the method comprises a subsequent steps of applying a conditioner composition and then performing a second rinse with water. Following these subsequent steps, the silicone that is deposited on the hair from the composition of the invention remains on the hair.

In a third aspect, the present invention provides a use of a hair substantive cationic conditioning polymer which is a homopolymer of (3-acrylamidopropyl) trimethyl ammonium chloride in shampoo to retain silicone on the hair.

Some or all of the silicone is retained on the hair following rinse with water, and/or following treatment with a hair conditioner and a second rinse. By "retained" on the hair is meant that the silicone remains on the hair.

### Detailed Description and Preferred Embodiments

### The Emulsified Silicone

The composition of the invention comprises a pre-formed emulsified silicone. Mixtures of emulsified silicones can be used.

Suitable silicones include polydiorganosiloxanes, in particular polydimethylsiloxanes which have the CTFA designation dimethicone. Also suitable for use compositions of the invention are polydimethyl siloxanes having hydroxyl end groups, which have the CTFA designation dimethiconol. Also suitable for use in compositions of the invention are silicone gums having a slight degree of cross-linking, as are described for example in WO 96/31188.

The viscosity of the emulsified silicone itself (not the emulsion or the final hair composition) is typically at least 10,000 cst at 25 °C the viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified silicones for use in the compositions of the invention will typically have a D90 silicone droplet size in the composition of less than 30, preferably less than 20, more preferably less than 10 micron, ideally from 0.01 to 1 micron. Silicone emulsions having an average silicone droplet size (D50) of 0.15 micron are generally termed microemulsions.

Silicone particle size may be measured by means of a laser light scattering technique, for example using a 2600D Particle Sizer from Malvern Instruments.

Examples of suitable pre-formed emulsions include Xiameter MEM 1785 and microemulsion DC2-1865 available from Dow Corning. These are emulsions /microemulsions of dimethiconol. Cross-linked silicone gums are also available in a pre-emulsified form, which is advantageous for ease of formulation.

A further preferred class of emulsified silicones for inclusion in compositions of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex Dow Corning).

Suitable quaternary silicone polymers are described in EP-A-0 530 974. A preferred quaternary silicone polymer is K3474, ex Goldschmidt.

Also suitable are emulsions of amino functional silicone oils with non ionic and/or cationic surfactant.

Pre-formed emulsions of amino functional silicone are also available from suppliers of silicone oils such as Dow Corning and General Electric. Specific examples include DC939

Cationic Emulsion and the non-ionic emulsions DC2-7224, DC2-8467, DC2-8177 and DC2-8154 (all ex Dow Corning).

Preferably, the silicone is selected from the group consisting of dimethicone, dimethiconol, amodimethicone and mixtures thereof. Also preferred are blends of amino-functionalised silicones with dimethicones.

The total amount of silicone is preferably from 0.01 wt% to 10 %wt of the total composition more preferably from 0.1 wt% to 5 wt%, most preferably 0.5 wt% to 3 wt% is a suitable level.

### The deposition polymer

The composition of the invention includes a cationic deposition polymers which may be selected from cationic polygalactomannans having a mean charge density at pH7 from 0.2 to 2 meq per gram. Such polymers may serve to enhance the delivery of conditioning agents from the composition to the skin and/or hair surface during consumer use, thereby improving the conditioning benefits obtained. Mixtures of cationic deposition polymers may be employed.

The term "charge density" in the context of this invention refers to the ratio of the number of positive charges on a monomeric unit of which a polymer is comprised to the molecular weight of the monomeric unit. The charge density multiplied by the polymer molecular weight determines the number of positively charged sites on a given polymer chain.

The polygalactomannans are polysaccharides composed principally of galactose and mannose units and are usually found in the endosperm of leguminous seeds, such as guar, locust bean, honey locust, flame tree, and the like. Guar flour is composed mostly of a galactomannan which is essentially a straight chain mannan with single membered galactose branches. The mannose units are linked in a 1-4-β-glycosidic linkage and the galactose branching takes place by means of a 1-6 linkage on alternate mannose units. The ratio of galactose to mannose in the guar polymer is therefore one to two.

Suitable cationic polygalactomannans for use in the invention include polygalactomannans, such as guars, and polygalactomannan derivatives, such as hydroxyalkyl guars (for example hydroxyethyl guars or hydroxypropyl guars), that have been cationically modified by chemical reaction with one or more derivatizing agents.

Derivatizing agents typically contain a reactive functional group, such as an epoxy group, a halide group, an ester group, an anhydride group or an ethylenically unsaturated group, and at least one cationic group such as a cationic nitrogen group, more typically a quaternary ammonium group. The derivatization reaction typically introduces lateral cationic groups on the polygalactomannan backbone, generally linked via ether bonds in which the oxygen atom corresponds to hydroxyl groups on the polygalactomannan backbone which have reacted.

Preferred cationic polygalactomannans for use in the invention include guar hydroxypropyltrimethylammonium chlorides.

Guar hydroxypropyltrimethylammonium chlorides for use in the invention are generally comprised of a nonionic guar gum backbone that is functionalized with ether-linked 2-hydroxypropyltrimethylammonium chloride groups, and are typically prepared by the reaction of guar gum with N-(3-chloro-2-hydroxypropyl) trimethylammonium chloride.

Cationic polygalactomannans for use in the invention (preferably guar hydroxypropyltrimethylammonium chlorides) generally have an average molecular weight (weight average molecular mass (Mw) determined by size exclusion chromatography) in the range 500,000 to 3 million g/mol, more preferably 800,000 to 2.5 million g/mol.

Cationic polygalactomannans for use in the invention generally have a charge density ranging from 0.5 to 1.8 meq/g.

Preferably the cationic polygalactomannans are selected from guar hydroxypropyltrimethylammonium chlorides having a charge density ranging from 0.5 to 1.8 meq/g (and mixtures thereof).

The cationic charge density of the polymer is suitably determined via the Kjeldahl method as described in the US Pharmacopoeia under chemical tests for nitrogen determination.

Specific examples of preferred cationic polygalactomannans are guar hydroxypropyltrimonium chlorides having a cationic charge density from 0.5 to 1.1 meq/g.

Also suitable are mixtures of cationic polygalactomannans in which one has a cationic charge density from 0.5 to 1.1 meq/g, and one has a cationic charge density from 1.1 to 1.8 meq per gram.

Specific examples of preferred mixtures of cationic polygalactomannans are mixtures of guar hydroxypropyltrimonium chlorides in which one has a cationic charge density from 0.5 to 1.1 meq/g, and one has a cationic charge density from 1.1 to 1.8 meq per gram.

Cationic polygalactomannans for use in the invention are commercially available from Rhodia as JAGUAR ^{®} C13S, JAGUAR ^{®} C14 and JAGUAR ^{®} C17.

In a typical composition according to the invention the level of cationic polygalactomannans will generally range from 0.05 to 2%, preferably from 0.1 to 0.5 , most preferably from 0.15 to 0.2% by weight based on the total weight of the composition.

In a preferred composition according to the invention the cationic polygalactomannans are selected from guar hydroxypropyltrimethylammonium chlorides having a charge density ranging from 0.5 to 1.8 meq/g (and mixtures thereof), at a level ranging from 0.15 to 0.2% by weight based on the total weight of the composition.

### The hair substantive cationic conditioning polymer

The composition of the invention includes a hair substantive cationic conditioning polymer which is a homopolymer of (3-acrylamidopropyl) trimethyl ammonium chloride.

WO2013/122861 describes the synthesis of (3-acrylamidopropyl) trimethyl ammonium chloride (APTAC) homopolymers of varying molecular weights, using a radical polymerisation reaction. According to the described method, APTAC monomer is polymerised in an aqueous medium by a discontinuous adiabatic process using an azo or persulfate radical initiator. The APTAC homopolymers so obtained have molecular weights ranging from about 100,000 g/mol to about 1,000,000 g/mol. The molecular weight can be determined by using standard analytical measurements, such as size exclusion chromatography (SEC).

A polymer suitable for use in the invention is commercially available from Ashland, Inc. as N-DurHance^{™} A-1000 Conditioning Polymer (supplied as a 20% a.i. aqueous solution of the polymer).

In a typical composition according to the invention the level of polymer (*per se* as active ingredient) generally ranges from 0.05 to 5 %, more preferably from 0.1 to 2%, most preferably from 0.15 to 1 % (by weight based on the total weight of the composition).

### The cleansing surfactant

The composition of the invention includes a cleansing surfactant. The cleansing surfactant may suitably be selected from one or more anionic surfactants.

Typical anionic surfactants for use as cleansing surfactants in the invention include those surface active agents which contain an organic hydrophobic group with from 8 to 14 carbon atoms, preferably from 10 to 14 carbon atoms in their molecular structure; and at least one water-solubilising group which is preferably selected from sulphate, sulphonate, sarcosinate and isethionate.

Specific examples of such anionic surfactants include ammonium lauryl sulphate, ammonium laureth sulphate, trimethylamine lauryl sulphate, trimethylamine laureth sulphate, triethanolamine lauryl sulphate, trimethylethanolamine laureth sulphate, monoethanolamine lauryl sulphate, monoethanolamine laureth sulphate, diethanolamine lauryl sulphate, diethanolamine laureth sulphate, lauric monoglyceride sodium sulphate, sodium lauryl sulphate, sodium laureth sulphate, potassium lauryl sulphate, potassium laureth sulphate, sodium lauryl sarcosinate, sodium lauroyl sarcosinate, lauryl sarcosine, ammonium cocoyl sulphate, ammonium lauroyl sulphate, sodium cocoyl sulphate, sodium lauryl sulphate, potassium cocoyl sulphate, potassium lauryl sulphate, monoethanolamine cocoyl sulphate, monoethanolamine lauryl sulphate, sodium tridecyl benzene sulphonate, sodium dodecyl benzene sulphonate, sodium cocoyl isethionate and mixtures thereof.

A preferred class of anionic surfactants for use as cleansing surfactants in the invention are alkyl ether sulphates of general formula:

R-O-(CH₂CH₂-O)ₙ-SO₃⁻M⁺

in which R is a straight or branched chain alkyl group having 10 to 14 carbon atoms, n is a number that represents the average degree of ethoxylation and ranges from 1 to 5, preferably from 1 to 3.5, and M is an alkali metal, ammonium or alkanolammonium cation, preferably sodium, potassium, monoethanolammonium or triethanolammonium, or a mixture thereof.

Specific examples of such preferred anionic surfactants include the sodium, potassium, ammonium or ethanolamine salts of C₁₀ to C₁₂ alkyl sulphates and C₁₀ to C₁₂ alkyl ether sulphates (for example sodium lauryl ether sulphate (nEO) in which n ranges from 1 to 3.5).

Mixtures of any of the above described materials may also be used.

In a typical composition of the invention the level of cleansing surfactant will generally range from 5 to 26% , and preferably ranges from 10 to 14% (by weight based on the total weight of the composition).

In a particularly preferred composition of the invention the cleansing surfactant is sodium lauryl ether sulphate (nEO) in which n ranges from 1 to 3.5, at a level of from 10 to 14% (by weight based on the total weight of the composition).

### The co- surfactant - CAPB

The composition of the invention includes a co-surfactant to shield the charge between the cationic polymer and the anionic surfactant. Preferably the co-surfactant is an amphoteric surfactant. Suitable amphoteric surfactants are betaines, such as those having the general formula R(CH₃)₂H⁺CH₂COO⁻, where R is an alkyl or alkylamidoalkyl group, the alkyl group preferably having 10 to 16 carbon atoms; and mixtures thereof. Particularly suitable betaines are oleyl betaine, caprylamidopropyl betaine, lauramidopropyl betaine, isostearylamidopropyl betaine, and cocoamidopropyl betaine and mixtures thereof.

The level of co-surfactant is generally from 0.1% to 20%, preferably from 1% to 10%, more preferably from 1% to 5% by weight based on the total weight of the composition.

### The Suspending agent

The composition of the invention includes one or more suspending agents. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

The suspending agent will generally be present in a shampoo composition for use in the invention at levels of from 0.1 to 10%, preferably from 0.15 to 6%, more preferably from 0.2 to 4% by total weight of suspending agent based on the total weight of the composition

### Water

The aqueous composition of the invention suitably comprises from about 50 to about 90%, preferably from about 55 to about 85%, more preferably from about 60 to about 85%, most preferably from about 65 to about 83% water (by weight based on the total weight of the composition).

### Salt

A preferred component of the composition is an inorganic electrolyte. Suitable inorganic electrolytes for use in the invention include metal chlorides (such as sodium chloride, potassium chloride, calcium chloride, magnesium chloride, zinc chloride, ferric chloride and aluminium chloride) and metal sulphates (such as sodium sulphate and magnesium sulphate). The inorganic electrolyte is used to assist in the solubilisation of oily components and to provide viscosity to the composition.

Examples of preferred inorganic electrolytes for use in the invention include sodium chloride, potassium chloride, magnesium sulphate and mixtures thereof.

Mixtures of any of the above described materials may also be suitable.

When included, the level of inorganic electrolyte in compositions of the invention generally ranges from about 1 to about 25%, preferably from about 1.5 to about 20% (by total weight inorganic electrolyte based on the total weight of the composition).

The composition of the invention may suitably have a viscosity ranging from 3,000 to 10,000 mPa.s, preferably from 4,000 to 9,000 mPa.s when measured using a Brookfield V2 viscometer (spindle RTV5, 1 minute, 20rpm) at 30°C.

### Other ingredients

A composition according to the invention may contain further optional ingredients to enhance performance and/or consumer acceptability. Examples of such ingredients include fragrance, dyes and pigments and pH adjusting agents. Each of these ingredients will be present in an amount effective to accomplish its purpose. Generally, these optional ingredients are included individually at a level of up to 5% by weight based on the total weight of the composition.

The pH of the composition of the invention suitably ranges from 3.0 to 7.0, and preferably ranges from 3.0 to 6.5, more preferably from 4 to 5.1.

The composition of the invention is primarily intended for topical application to the hair and scalp.

Most preferably the composition of the invention is topically applied to the hair and then massaged into the hair and scalp. The composition is then rinsed off the hair and scalp with water prior to drying the hair.

The invention will be further illustrated by the following, non-limiting Examples.

### EXAMPLES

### Example 1: Compositions 1-2, in accordance with the invention. Comparative Compositions A-B and Control 1

Hair cleansing shampoo formulations were prepared, having ingredients as shown in Table 1. Compositions 1-2 are in accordance with the invention; Compositions A-B are comparative examples. All weight percentages (wt%) quoted are by weight based on total weight unless otherwise stated.

**Table 1: Compositions (wt %) of Compositions 1-2, in accordance with the invention, Comparative Compositions A-B and Control 1**

| **INGREDIENT** | **CONTROL 1** | **COMPOS ITION A** | **COMPOS ITION 1** | **COMPOS ITION B** | **COMPOS ITION 2** |
|---|---|---|---|---|---|
| | wt% | | | | |
| Sodium laureth sulphate (1EO) | 14 | 12 | 12 | 12 | 12 |
| Cocamidopropyl betaine (CAPB) | 1.6 | 1.6 | 1.6 | 1.6 | 1.6 |
| Guar hydroxypropyl trimonium chloride | - | 0.2 | 0.2 | 0.2 | 0.2 |
| Dimethiconol* | - | 1 | 1 | - | - |
| DC7051** | - | - | - | 1 | 1 |
| Carbomer | - | 0.4 | 0.4 | 0.4 | 0.4 |
| Cationic conditioning polymer *** | - | - | 1.0 | - | 1.0 |
| Sodium chloride | 0.4 | 0.2 | 0.2 | 0.2 | 0.2 |
| Water, and minors (perfume, preservatives, pH adjusters) | to 100% | to 100% | to 100% | to 100% | to 100% |

| | | | | | |
|---|---|---|---|---|---|
| * Emulsion of dimethiconol with anionic emulsifier, average particle size < 1 micron (ex Dow) ** Dowsil CE-7051 POE Emulsion silicone blend ex Dow *** N-DurHance^{™} A-1000 conditioning polymer (ex Ashland Inc.); 20 % active | | | | | |

The shampoos Compositions 1-2, Comparative Compositions A-B and the Control were prepared by the following method:
1. The cationic conditioning polymer was thoroughly dispersed in water.
2. The CAPB was then added.
3. The cleansing surfactant was added to the aqueous mixture and fully dispersed.
4. The suspending agent (carbomer) was added.
5. The guar polymer was dispersed in water and added to the mixture.
6. The silicone was then added with stirring and
7. The remaining minor ingredients were added.
8. Finally, the pH and viscosity of the shampoo were adjusted using pH adjuster (for example, citric acid) and sodium chloride respectively.

### Example 2: Treatment of hair with Compositions 1-2, Comparative Compositions A-B and Control 1

The hair used was dark brown European hair, in switches of 2.5 g weight and 6 inch length. This is referred to in these examples as Virgin hair.

Bleached hair was prepared as follows:
Hair was bleached once for 30 min with Platine Precision White Compact Lightening Powder (L'Oreal Professionnel Paris, Paris, France) mixed with 9% cream peroxide, 30 'vol' (Excel GS Ltd, UK) (60g of powder mixed with 120g cream peroxide). Hair was then rinsed with water for 2 minutes.

The formulations described in Table 1 were used to treat the hair during a typical washing protocol. The treated hair was then assessed for the level of silicone deposited onto the surface as well as the level of friction when the hair was dry.

Hair was treated with shampoo compositions Compositions 1-2, Comparative Compositions A-B and Control 1 using the following method:-

The hair fibres were held under running water for 30 seconds, shampoo applied at a dose of 0.1 ml of shampoo per 1g of hair and rubbed into the hair for 30 seconds. Excess lather was removed by holding under running water for 30 seconds and the shampoo stage repeated. The hair was rinsed under running water for 1 minute.

Hair was then treated with a silicone free conditioner where 0.2 ml conditioner per gram hair was applied and rubbed in for 1 min, then rinsed for 1 min under running water.

### Example 3: Silicone Deposition onto hair treated with Compositions 1-2, Comparative Compositions A-B and Control 1

Treated hair switches were rinsed and dried before the level of silicone deposited on the hair surface was quantified using x-ray fluorescence (XRF).

Five replicas were produced for each test formulation. The average amount of silicone deposited onto hair is shown in Table 2.

**Table 2: Deposition on silicone (ppm) onto bleached hair treated with Compositions 1-2, in accordance with the invention, Comparative Compositions A-B and Control 1**

| **Composition** | **Hair type** | **Silicone Deposition (ppm)** | **s.d.** |
|---|---|---|---|
| Control 1 | bleached | 0 | 10.07 |
| Composition A | bleached | 30.32 | 4.52 |
| Composition 1 | bleached | 164.06 | 17.54 |
| Composition B | virgin | 408.6 | 39.46 |
| Composition 2 | virgin | 650.6 | 39.84 |

It will be seen that the level of silicone deposited onto hair is dramatically higher for Compositions 1 and 2, in accordance with the invention, than for the comparative Compositions A and B, which did not comprise the polymer. Unexpectedly, the silicone delivered from the shampoo was retained through the conditioner application and rinse process.

### Example 4: Friction of hair treated with Compositions 1-2, Comparative Compositions A-B and Control 1

In a further series of tests, formulations described in Table 1 were assessed for the level of friction following a typical hair washing protocol on switches of virgin or once-bleached dark brown European (DBE) hair. Five replicas were produced for each test formulation. The average of measured friction is shown in Table 3.

Hair was first washed with shampoo and conditioner as in Example 2 above.

The friction of the dry hair was assessed using a Texture Analyser fitted with a 500 g weight on top of the probe.

**Table 3: Friction of bleached hair treated with Compositions 1-2, in accordance with the invention, Comparative Compositions A-B and Control 1**

| **Formulation** | **Dry Friction (mm.g)** | **s.d.** |
|---|---|---|
| Control 1 | 52837 | 3347 |
| Composition A | 48622 | 1310 |
| Composition 1 | 34361 | 1148 |
| Composition B | 26885 | 791 |
| Composition 2 | 23139 | 628 |

It can be seen that hair treated with Compositions in accordance with the invention produces less friction than hair treated with Comparative Compositions A and B.

## Claims

1. An aqueous shampoo composition comprising:-
a. a pre-formed emulsified silicone;
b. a cationic deposition polymer;
c. a hair substantive cationic conditioning polymer which is a homopolymer of (3-acrylamidopropyl) trimethyl ammonium chloride;
d. a cleansing surfactant;
e. a co-surfactant; and
f. a suspending agent.

2. A composition as claimed in claim 1, wherein the amount of emulsified silicone is from 0.01 to 10 wt % by total weight 100 % active silicone based on the total weight of the composition.

3. A composition as claimed in claim 1 or claim 2, wherein the the emulsified silicone is selected from the group consisting of dimethicone, dimethiconol, amodimethicone and mixtures thereof.

4. A composition as claimed in claim 1 or claim 2, wherein the the emulsified silicone emulsion is a blend of an amino-functionalised silicone with a dimethicone.

5. A composition as claimed in any preceding claim, wherein the co-surfactant is an amphoteric surfactant selected from betaines, preferably having the general formula R(CH₃)₂N⁺CH₂COO⁻, where R is an alkyl or alkylamidoalkyl group, the alkyl group preferably having 10 to 16 carbon atoms; and mixtures thereof.

6. A composition as claimed in any preceding claim, wherein the co-surfactant is present in an amount of from 0.1 % to 20%, by weight based on the total weight of the composition.

7. A composition according to any preceding claim, in which the deposition polymer(s) is a cationic polygalactomannan selected from guar hydroxypropyltrimethylammonium chlorides with a charge density ranging from 0.5 to 1.8 meq/g (and mixtures thereof).

8. A composition according to claim 7, in which the level of the guar hydroxypropyltrimethylammonium chloride(s) ranges from 0.15 to 0.2% by weight based on the total weight of the composition.

9. A composition according to any preceding claim, in which the level of polymer (*per se* as active ingredient) ranges from 0.2 to 1.5% (by weight based on the total weight of the composition).

10. A composition as claimed in any preceding claim, wherein the suspending agent is selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives.

11. A composition as claimed in any preceding claim, wherein the suspending agent is present at levels of from 0.1 to 10 wt % by total weight of suspending agent based on the total weight of the composition.

12. A method of treating hair comprising the steps of applying to hair the composition as defined in any one of claims 1 to 11, and performing a first rinse with water.

13. A method as claimed in claim 12, which comprises the subsequent steps of applying a conditioner composition and performing a second rinse with water.

14. A use of a hair substantive cationic conditioning polymer which is a homopolymer of (3-acrylamidopropyl) trimethyl ammonium chloride in shampoo to retain silicone on the hair.

## Patentansprüche

1. Wässrige Shampoo-Zusammensetzung, umfassend:
a. ein vorgeformtes emulgiertes Silikon;
b. ein kationisches Ablagerungspolymer;
c. ein Haar-substanzielles kationisches Konditionierungspolymer, das ein Homopolymer vom (3-Acrylamidopropyl)trimethylammoniumchlorid ist;
d. ein reinigendes Tensid;
e. ein Co-Tensid; und
f. ein Suspendiermittel.

2. Zusammensetzung, wie im Anspruch 1 beansprucht, wobei die Menge des emulgierten Silikons von 0,01 bis 10 Gew.-%, bezogen auf das Gesamtgewicht von 100% aktivem Silikon, bezogen auf das Gesamtgewicht der Zusammensetzung, beträgt.

3. Zusammensetzung, wie im Anspruch 1 oder Anspruch 2 beansprucht, wobei das emulgierte Silikon aus der Gruppe ausgewählt ist, bestehend aus Dimethicone, Dimethiconol, Amodimethicone und Mischungen davon.

4. Zusammensetzung, wie im Anspruch 1 oder Anspruch 2 beansprucht, wobei die emulgierte Silikonemulsion eine Mischung eines amino-funktionalisierten Silikons mit einem Dimethicone ist.

5. Zusammensetzung, wie in einem vorhergehenden Anspruch beansprucht, wobei das Co-Tensid ein amphoteres Tensid ist, ausgewählt aus Betainen, die vorzugsweise die allgemeine Formel R(CH₃)₂N⁺CH₂COO⁻ aufweisen, wobei R eine Alkyl- oder Alkylamidoalkylgruppe ist, wobei die Alkylgruppe vorzugsweise 10 bis 16 Kohlenstoffatome aufweist, und Mischungen davon.

6. Zusammensetzung, wie in einem vorhergehenden Anspruch beansprucht, wobei das Co-Tensid in einer Menge von 0,1 bis 20 Gewichts-%, bezogen auf das Gesamtgewicht der Zusammensetzung, vorliegt.

7. Zusammensetzung nach einem vorhergehenden Anspruch, in der das (die) Ablagerungspolymer(e) ein kationisches Polygalactomannan ist (sind), ausgewählt aus Guar-Hydroxypropyltrimethylammoniumchloriden mit einer Ladungsdichte im Bereich von 0,5 bis 1,8 mäq/g (und Mischungen davon).

8. Zusammensetzung nach Anspruch 7, in der der Gehalt an Guar-Hydroxypropy-Itrimethylammonoiumchlorid(en) in dem Bereich von 0,15 bis 0,2 Gewichts-%, bezogen auf das Gesamtgewicht der Zusammensetzung, liegt.

9. Zusammensetzung nach einem vorhergehenden Anspruch, in der der Gehalt des Polymers (per se als aktiver Bestandteil) in dem Bereich von 0,2 bis 1,5 Gew.-% (bezogen auf das Gesamtgewicht der Zusammensetzung) liegt.

10. Zusammensetzung, wie in einem vorhergehenden Anspruch beansprucht, wobei das Suspendiermittel ausgewählt ist aus Polyacrylsäuren, vernetzten Polymeren von Acrylsäure, Copolymeren von Acrylsäure mit einem hydrophoben Monomer, Copolymeren von carbonsäurehaltigen Monomeren und Acrylestern, vernetzten Copolymeren von Acrylsäure und Acrylatestern, Heteropolysaccharidgummis und kristallinen langkettigen Acylderivaten.

11. Zusammensetzung, wie in einem vorhergehenden Anspruch beansprucht, wobei das Suspendiermittel in Mengen von 0,1 bis 10 Gew.-% des Gesamtgewichts des Suspendiermittels, bezogen auf das Gesamtgewicht der Zusammensetzung, vorliegt.

12. Verfahren zur Haarbehandlung, umfassend die Schritte des Auftragens der Zusammensetzung auf das Haar, wie in irgendeinem der Ansprüche 1 bis 11 definiert, und Durchführen einer ersten Spülung mit Wasser.

13. Verfahren, wie im Anspruch 12 beansprucht, das die aufeinanderfolgenden Schritte des Auftragens einer Konditionierungszusammensetzung und des Durchführens einer zweiten Spülung mit Wasser umfasst.

14. Verwendung eines Haar-substanziellen kationischen Konditionierungspolymers, das ein Homopolymer von (3-Acrylamidopropyl)trimethylammoniumchlorid, in einem Shampoo ist, um Silikon auf dem Haar zurückzuhalten.

## Revendications

1. Composition aqueuse de shampoing comprenant :
a. un silicone émulsifié pré-formé ;
b. un polymère de dépôt cationique ;
c. un polymère de conditionnement cationique substantif pour cheveux qui est un homopolymère de chlorure de (3-acrylamidopropyl)triméthylammonium ;
d. un tensioactif nettoyant ;
e. un co-tensioactif ; et
f. un agent de mise en suspension.

2. Composition selon la revendication 1, dans laquelle la quantité de silicone émulsifié est de 0,01 à 10 % en masse en masse totale de 100 % de silicone actif sur la base de la masse totale de la composition.

3. Composition selon la revendication 1 ou revendication 2, dans laquelle le silicone émulsifié est choisi dans le groupe consistant en diméthicone, diméthiconol, amodiméthicone et des mélanges de ceux-ci.

4. Composition selon la revendication 1 ou revendication 2, dans laquelle l'émulsion de silicone émulsifié est une combinaison d'un silicone amino-fonctionnalisé avec une diméthicone.

5. Composition selon l'une quelconque des revendications précédentes, dans laquelle le co-tensioactif est un tensioactif amphotère choisi parmi des bétaïnes, ayant de préférence la formule générale R(CH₃)₂N⁺CH₂COO⁻, où R est un groupe alkyle ou alkylamidoalkyle, le groupe alkyle ayant de préférence de 10 à 16 atomes de carbone ; et des mélanges de ceux-ci.

6. Composition selon l'une quelconque des revendications précédentes, dans laquelle le co-tensioactif est présent dans une quantité de 0,1 % à 20 %, en masse sur la base de la masse totale de la composition.

7. Composition selon l'une quelconque des revendications précédentes, dans laquelle le(les) polymère(s) de dépôt est(sont) un polygalactomannane cationique choisi parmi des chlorures de guar hydroxypropyltriméthylammonium avec une densité de charge de 0,5 à 1,8 meq/g (et mélanges de ceux-ci).

8. Composition selon la revendication 7, dans laquelle la teneur du(des) chlorure(s) de guar hydroxypropyltriméthylammonium est de 0,15 à 0,2 % en masse sur la base de la masse totale de la composition.

9. Composition selon l'une quelconque des revendications précédentes, dans laquelle la teneur de polymère (*per se* comme ingrédient actif) est de 0,2 à 1,5 % (en masse sur la base de la masse totale de la composition).

10. Composition selon l'une quelconque des revendications précédentes, dans laquelle l'agent de mise en suspension est choisi parmi des poly(acides acryliques), polymères réticulés d'acide acrylique, copolymères d'acide acrylique avec un monomère hydrophobe, copolymères de monomères contenant de l'acide carboxylique et d'esters acryliques, copolymères réticulés d'acide acrylique et d'esters d'acrylate, gommes d'hétéropolysaccharides et dérivés acyliques à chaîne longue cristallins.

11. Composition selon l'une quelconque des revendications précédentes, dans laquelle l'agent de mise en suspension est présent à des teneurs de 0,1 à 10 % en masse en masse totale de l'agent de mise en suspension sur la base de la masse totale de la composition.

12. Procédé de traitement des cheveux comprenant les étapes d'application aux cheveux de la composition selon l'une quelconque des revendications 1 à 11, et la réalisation d'un premier rinçage avec de l'eau.

13. Procédé selon la revendication 12, qui comprend les étapes subséquentes d'application d'une composition de conditionnement et de réalisation d'un second rinçage avec de l'eau.

14. Utilisation d'un polymère de conditionnement cationique substantif pour cheveux qui est un homopolymère de chlorure de (3-acrylamidopropyl)triméthyl ammonium dans un shampoing pour retenir du silicone sur les cheveux.
